**Europäisches Patentamt**

**European Patent Office**

**Office européen des brevets**

(19)

(11) Veröffentlichungsnummer : **0 476 359 B1**

## (12) EUROPÄISCHE PATENTSCHRIFT

(45) Veröffentlichungstag der Patentschrift :
**29.09.93 Patentblatt 93/39**

(51) Int. Cl.$^5$ : **C07C 209/72, B01J 23/46**

(21) Anmeldenummer : **91114225.5**

(22) Anmeldetag : **24.08.91**

(54) **Verfahren zur Herstellung von Di-(4-aminocyclohexyl)-methan mit einem Anteil an 15 bis 25 Gew.-% des trans-trans-Isomeren.**

(30) Priorität : **06.09.90 DE 4028270**

(43) Veröffentlichungstag der Anmeldung :
**25.03.92 Patentblatt 92/13**

(45) Bekanntmachung des Hinweises auf die
Patenterteilung :
**29.09.93 Patentblatt 93/39**

(84) Benannte Vertragsstaaten :
**DE FR GB IT**

(56) Entgegenhaltungen :
**EP-A- 0 053 819
EP-A- 0 324 190
EP-A- 0 351 661
DE-A- 1 542 392
DE-A- 1 593 293
US-A- 3 644 522**

(73) Patentinhaber : **BAYER AG
D-51368 Leverkusen (DE)**

(72) Erfinder : **Immel, Otto, Dr.
Immenhofweg 26
W-4150 Krefeld (DE)**
Erfinder : **Darsow, Gerhard, Dr.
Zu den Tannen 39
W-4150 Krefeld (DE)**
Erfinder : **Braden, Rudolf, Dr.
Nothauserfeld 1
W-5068 Odenthal (DE)**
Erfinder : **Schwarz, Hans-Helmut, Dr.
Rather Strasse 90
W-4150 Krefeld 1 (DE)**
Erfinder : **Waldmann, Helmut, Dr.
Henry-Th.-v.-Böttinger-Strasse 15
W-5090 Leverkusen 1 (DE)**

## Beschreibung

Gegenstand der Erfindung ist ein verbessertes Verfahren zur Herstellung eines flüssigen Gemisches der Isomeren von Di-(4-aminocyclohexyl)-methan durch Hydrierung von Di-(4-aminophenyl)-methan (MDA). Ein solches erfindungsgemäß herstellbares flüssiges Isomerengemisch enthält 15 bis 25 Gew.-%, bevorzugt 18 bis 23,5 Gew.-%, des trans-trans-Isomeren.

Ein flüssiges Isomerengemisch ist vor allen Dingen gefragt zur Herstellung eines seinerseits bei Raumtemperatur (etwa 10 bis 25°C) flüssigen, hiervon abgeleiteten Diisocyanats.

Im allgemeinen führt die Hydrierung von MDA zu einem Isomerengemisch, in welchem das cis-cis-Isomere, das cis-trans-Isomere und das trans-trans-Isomere gemeinsam vorkommen und in dem der trans-trans-Anteil der Gleichgewichtskonzentration von etwa 50 Gew.-% zustrebt. Durch ein aufwendiges Umwandlungsverfahren gemäß DE-AS 1 593 293 kann man die gewünschte Isomerenzusammensetzung mit dem obengenannten niedrigeren trans-trans-Isomerenanteil erreichen. Es sind noch weitere Verfahren bekannt geworden, die einen bestimmten trans-trans-Anteil im Isomerengemisch des Di-(4-aminocyclohexyl)-methans einzustellen gestatten (US 3.766.272; US 3.644.522; US 3.155.724; US 3.153.088). In EP 324 190 ist auch bereits ein Hydrierverfahren für MDA beschrieben worden, das durch Verwendung besonderer Katalysatoren und unter Einhaltung bestimmter Reaktionsbedingungen Hydrierprodukte erzeugt, deren trans-trans-Anteil dem gewünschten Wert bereits entspricht. Dieses zuletzt genannte Verfahren hat jedoch den Nachteil, daß die Gesamtausbeute an Di-(4-aminocyclohexyl)-methan unbefriedigend ist. Weiterhin ist bei einigen der genannten Hydrier- bzw. Umwandlungsverfahren die Mitverwendung von Ammoniak oder von hochwertigen Lösungsmitteln erforderlich.

Es bestand daher nach wie vor die Aufgabe, ein neues, vereinfachtes und damit auch in technischem Maßstab brauchbares Verfahren zu entwickeln, in welchem bei hohem Gesamtumsatz der trans-trans-Isomerengehalt im obengenannten gewünschten Bereich liegt. Diese Aufgabe kann durch das erfindungsgemäße Verfahren gelöst werden.

Es wurde ein Verfahren zur Herstellung von Di-(4-aminocyclohexyl)-methan mit einem Anteil von 15 bis 25 Gew.-% des trans-trans-Isomeren durch katalytische Hydrierung von Di-(4-aminophenyl)-methan bei erhöhter Temperatur und erhöhtem Wasserstoffdruck in Gegenwart eines Ruthenium enthaltenden Trägerkatalysators gefunden, das dadurch gekennzeichnet ist, daß man einen Ruthenium-Katalysator auf einem mit Verbindungen von seltenen Erdmetallen (SE) und von Mangan behandelten $Al_2O_3$-Träger mit Rutheniumgehalten von 0,05 bis 5 Gew.-%, bevorzugt 0,05 bis 3 Gew.-%, besonders bevorzugt 0,1 bis 2 Gew.-%, bezogen auf das Gesamtgewicht des Katalysators, einsetzt.

Das erfindungsgemäße Verfahren ist demnach durch den Einsatz eines speziellen Ruthenium-Trägerkatalysators gekennzeichnet, dessen Träger $Al_2O_3$ in der obengenannten speziellen weise Verbindungen der SE und des Mangans in den angegebenen Mengen enthält (Solche Katalysatoren sind bereits aus EP-A-351661 zur Hydrierung von Anilin bekannt). Zur weiteren Verbesserung führt ein Zusatz basischer Alkaliverbindungen.

Als $Al_2O_3$ wird besonders die α- oder die γ-Modifikation, in bevorzugter Weise die γ-Modifikation, eingesetzt.

Der Gehalt an SE und Mangan beträgt zusammen 0,05 bis 8 Gew.-%, bevorzugt 0,2 bis 5 Gew.-%, bezogen auf das Gesamtgewicht des Katalysators. Das Gewichtsverhältnis von SE zu Mangan beträgt 5:1 bis 1:5, bevorzugt 10:9 bis 1:2.

Als SE werden die Elemente der III. Nebengruppe des Periodensystems (Mendelejew), wie Scandium, Yttrium, Lanthan und die Lanthaniden verstanden. Als SE kann sowohl eines der genannten Elemente als auch ein Gemisch mehrerer von ihnen eingesetzt werden. Dies ist insbesondere deshalb wichtig, weil auch rohe Gemische von SE, wie sie technisch verfügbar sind, und in denen zunächst nur eines oder zwei der SE angereichert sind, eingesetzt werden können. In bevorzugter Weise wird eines oder mehrere der Elemente aus der Gruppe Yttrium, Lanthan, Cer, Praseodym, Neodym und Dysprosium eingesetzt. In besonders bevorzugter Weise wird Cer und/oder Lanthan singesetzt. In ganz besonderer Weise wird Cer, gegebenenfalls in einem an Cer angereichertem Gemisch singesetzt. Zur Beaufschlagung des $Al_2O_3$-Trägers liegen die SE und Mangan in Form ihrer Verbindungen, bevorzugt in oxidischer Form, vor.

Als basische Zusätze können die Oxide, Hydroxide oder Carbonate der Alkalimetalle, bevorzugt NaOH und KOH, dienen. Dabei können die basischen Zusätze vor oder nach der Metallbeschichtung auf den Katalysatorträger gebracht werden. Auf das Gewicht des Katalysators bezogen, beträgt der Alkalimetallzusatz 0,1 bis 10 %, bevorzugt 0,2 bis 5 %.

Zur Herstellung des erfindungsgemäß einsetzbaren Katalysators kann beispielsweise so vorgegangen werden, daß auf ein $Al_2O_3$ in Form von Strangpreßlingen, Pillen oder Kugeln mit Abmessungen von etwa 2 bis 10 mm Verbindungen der SE und des Mangans aufgebracht werden; der so beaufschlagte Träger wird getrocknet und dann weiter mit einem Rutheniumsalz behandelt, wonach sich eine erneute Trocknungsphase anschließt. In bevorzugter Weise wird der zunächst mit SE und Mangan beaufschlagte Katalysatorträger auf eine Temperatur von 200 bis 450°C erhitzt, bevor das Ruthenium aufgetragen wird.

Das Aufbringen von Verbindungen der SE und des Mangans kann beispielsweise durch Tränken oder Sprühen mit geeigneten Salzen der genannten Elemente erfolgen. Durch eine geeignete Trockentemperatur, insbesondere durch das bevorzugte Erhitzen auf 200 bis 450°C, werden die Salze der SE und des Mangans in auf dem Katalysatorträger fest haftende Verbindungen übergeführt. Das Aufbringen von Verbindungen der SE und des Mangans kann jedoch auch durch gemeinsames Ausfällen eines SE-Mangan-Hydroxid-Gemisches aus SE- und Mangan-Salzen auf dem getränkten Träger mit Alkalilauge oder Ammoniak und gegebenenfalls anschließendes Auswaschen der löslichen Anteile mit Wasser erfolgen. Als Salze der SE und des Mangans kommen insbesondere die Sulfate, die Chloride, die Acetate und/oder die Nitrate der genannten Elemente in Betracht. Der mit SE und Mangan behandelte Träger wird zunächst getrocknet und dann in bevorzugter Weise während einer Zeit von 1 bis 120 Stunden auf 200 bis 450°C, bevorzugt 250 bis 430°C, erhitzt. Während der genannten Zeit kann die Temperatur im angegebenen Bereich von niederen auf höhere Werte erhöht werden.

Zur anschließend erfolgenden Tränkung des so behandelten Trägers mit Ruthenium kann so vorgegangen werden, daß das Ruthenium, beispielsweise in Form einer wäßrigen Lösung des Chlorids, Nitrats, Acetats oder eines anderen geeigneten Salzes auf den Träger aufgetränkt oder aufgesprüht wird, gefolgt wiederum von einer Trocknung. Man kann jedoch auch vor der Trocknung den mit Ruthenium getränkten Träger mit einer Lösung der obengenannten basischen Verbindungen behandeln, wobei das Ruthenium als Oxid oder Hydroxid ausfällt. Hier kann sich eine Auswaschung der wasserlöslichen Anteile und wiederum eine Trocknung anschließen. Danach steht ein solcher erfindungsgemäß einsetzbarer Katalysator grundsätzlich zum Einsatz zur Verfügung. In bevorzugter Weise wird er jedoch vor seinem Einsatz, besonders bevorzugt nach Anordnung im Hydrierreaktor, durch Behandlung mit Wasserstoff bei einer Temperatur von 150 bis 350°C aktiviert. Auch nach einer solchen Aktivierung kann es wünschenswert sein, Anionen wie Chlorid, Nitrat, Acetat oder andere, und gegebenenfalls die Kationen der zur Ausfällung benutzten basischen Verbindungen durch eine Wasserwäsche zu entfernen. Man kann jedoch auch den mit Verbindungen der SE und des Mangans beaufschlagten Katalysatorträger zunächst mit der Lösung einer der genannten basischen Verbindungen tränken, anschließend trocknen und auf den so vorbehandelten, basisch eingestellten Katalysatorträger Lösungen von Rutheniumsalzen auftragen, wobei im Moment der Tränkung auch die Ausfällung des Rutheniums in Form seines Oxids oder Hydroxids erfolgt. Auch hierbei ist nach einer abschließenden Trocknung der Katalysator grundsätzlich einsatzbereit, kann jedoch bevorzugt in der beschriebenen Weise vorab mit Wasserstoff bei der genannten Temperatur aktiviert werden. Auch bei dieser Variante ist der Ruthenium-Trägerkatalysator grundsätzlich in Gegenwart der Reste solcher alkalischer Verbindungen betriebsbereit. In bevorzugter Weise wird jedoch die beschriebene Wasserwäsche vorgenommen.

Das Tränken oder das Besprühen des $Al_2O_3$-Trägers mit den genannten Stoffen und die hierfür erforderlichen Arbeitsgeräte sind dem Fachmann bekannt; ebenso bekannt ist die Einstellung der gewünschten Beaufschlagung durch die Wahl der Menge und Konzentration der Lösungen der genannten Elemente.

Die erfindungsgemäße Hydrierung erfolgt in einem Temperaturbereich von 80 bis 160°C, bevorzugt 90 bis 140°C.

Dabei richtet sich die Wahl der Temperatur nach der gewünschten Reaktionsgeschwindigkeit sowie nach der angestrebten Zusammensetzung des Isomerengemisches.

Mit steigender Temperatur wächst die Reaktionsgeschwindigkeit, aber auch der trans-trans-Anteil im entstehenden Isomerengemisch. Auf das Hydrierprodukt wirkt der Katalysator, besonders bei höheren Temperaturen, isomerisierend ein, so daß der trans-trans-Anteil bei längerer Kontaktzeit noch weiter ansteigen kann. Daher ist es im allgemeinen vorteilhaft, das Hydrierprodukt nach beendeter Wasserstoffaufnahme vom Katalysator zu trennen.

Erfindungsgemäß liegt der angewandte Wasserstoffdruck bei 20 bis 500 bar, vorzugsweise bei 200 bis 400 bar. Je höher der Wasserstoffdruck gewählt wird, desto schneller verläuft die Hydrierung. Bei höherem Wasserstoffdruck ist es somit möglich, eine niedrigere Hydriertemperatur und/oder eine kürzere Kontaktzeit mit dem Katalysator einzuhalten, um den trans-trans-Anteil im Hydrierprodukt zu verringern.

Bei diskontinuierlicher Hydrierung im Autoklaven kann man den Ablauf der Reaktion an der aufgenommenen Wasserstoffmenge verfolgen und damit das Ende der Hydrierzeit erkennen. Die Hydrierzeit beträgt in Abhängigkeit von der Katalysatormenge und der eingestellten Temperatur im oben beschriebenen Sinne 2 bis 5 Stunden.

Besonders vorteilhaft läßt sich das erfindungsgemäße Verfahren kontinuierlich, etwa in der Rieselphase, durchführen. Hierzu wird der Katalysator in Form eines Granulats in ein senkrecht angeordnetes Druckrohr gefüllt. Das zu hydrierende MDA, gegebenenfalls gemeinsam mit einem inerten Lösungsmittel zur Verdünnung, und der Wasserstoff werden von oben her über die Katalysatorschüttung geleitet. Der vom ausgetragenen Reaktionsprodukt abgetrennte Wasserstoff kann erneut für die Umsetzung verwendet werden. Die günstigste Durchsatzgeschwindigkeit läßt sich bei gegebenen Reaktionsbedingungen durch eine gaschromatographische Analyse (GC-Analyse) des Hydrierproduktes einstellen und verfolgen. Eine Katalysatorbelastung von 0,05 bis 0,5 kg MDA je Liter Katalysator und Stunde hat sich als vorteilhaft erwiesen.

Als inertes Verdünnungsmittel eignen sich flüssige Verbindungen, wie Dioxan, Tetrahydrofuran, Isobutanol oder tert.-Butanol, die dem Fachmann als hydrierinert bekannt sind. Zur Vermeidung eines systemfremden Verdünnungsmittels kann jedoch in bevorzugter Weise ein Teil des entstehenden Hydrierproduktes zur Verdünnung eingesetzt werden. Bei der Verwendung eines inerten Verdünnungs-/Lösungsmittels beziehungsweise des entstandenen Hydrierproduktes als Verdünnung beträgt im allgemeinen das Verhältnis von MDA zu Verdünnungsmittel 1:0,2 bis 1:2, bevorzugt 1:0,5 bis 1:1.

Im Vergleich zu den Verfahren gemäß Stand der Technik zeigt das erfindungsgemäße Verfahren den Vorteil der höheren Ausbeute in Verbindung mit dem unmittelbaren Erhalt des gewünschten Isomerenverhältnisses und unter Verwendung eines einfach herstellbaren Ruthenium-Katalysators.

In den folgenden Beispielen beziehen sich sämtliche Angaben von Prozentsätzen auf das Gewicht.

## Beispiele

### Beispiel 1

200 g eines handelsüblichen $\gamma$-$Al_2O_3$ mit einer spezifischen Oberfläche von 350 m²/g und einem Kugeldurchmesser von 2 bis 6 mm wurden mit einer Lösung getränkt, die aus 12,4 g $Ce(NO_3)_3 \cdot 6\ H_2O$, 18,28 g $Mn(NO_3)_2 \cdot 4\ H_2O$ und 50 g Wasser hergestellt worden war, Das getränkte $Al_2O_3$ wurde im Wasserstrahlvakuum 18 Stunden bei 120°C getrocknet und anschließend 3 Stunden lang bei 400°C getempert. Der so hergestellte Katalysatorträger wurde mit 70 g einer wäßrigen $RuCl_3$-Lösung getränkt, die 2 g Ru enthielt, Der feuchte Katalysator wurde im Wasserstrahlvakuum 18 Stunden bei 120°C getrocknet und 3 Stunden im Wasserstoffstrom (100 l $H_2$/h) bei 350°C aktiviert.

### Beispiel 2

25 ml (19 g) des Ru-Ce-Mn-$Al_2O_3$-Katalysators, der nach Beispiel 1 hergestellt wurde und 1 % Ru, 2 % Ce und 2 % Mn enthielt, wurde zur Hydrierung von Di-(4-aminophenyl)-methan (MDA) in einem 0,25 l-Schüttelautoklaven verwendet. Der Autoklav war im Inneren mit einem Siebkorb ausgestattet, in den der Katalysator gefüllt wurde.

Mit dieser Katalysatorfüllung wurden sechsmal nacheinander 40 g MDA, gelöst in 40 tert.-Butanol, hydriert. Der Wasserstoffdruck betrug 260 bis 300 bar und die Reaktionstemperatur 140°C. Die erforderliche Hydrierzeit lag zwischen 240 bis 300 min.. Nach den einzelnen Hydrierungen wurde der Autoklav auf Raumtemperatur abgekühlt; das Reaktionsprodukt wurde entnommen, und der Autoklav wurde wieder mit einer Lösung von MDA in tert.-Butanol gefüllt. Die Reaktionsprodukte wurden auf ihren Gehalt an trans-trans-Isomerem und perhydriertem (12 zusätzliche H-Atome) MDA analysiert. Die erhaltenen Ergebnisse sind in der folgenden Tabelle aufgeführt:

| Hydrierzeit (Min.) | trans-trans- Isomeres (%) | 3- und 4-Kern- Verbindungen (%) | 12-HMDA (%) |
|---|---|---|---|
| 300 | 22,5 | 2,8 | 96,5 |
| 265 | 19,8 | 2,6 | 96,9 |
| 240 | 19,0 | 1,7 | 97,9 |
| 270 | 21,0 | 1,5 | 98,3 |
| 260 | 20,6 | 1,9 | 97,7 |

### Beispiel 3

150 g des nach Beispiel 1 hergestellten Katalysators wurden zur Nachbehandlung mit einer Lösung getränkt, die aus 54 g Wasser und 6 g NaOH hergestellt worden war. Die Menge der eingesetzten Natronlauge entsprach der Saugfähigkeit des Katalysators. Der getränkte Katalysator wurde 20 Stunden bei 100°C getrocknet. 60 ml (53 g) des so hergestellten Katalysators wurden in ein senkrecht angeordnetes Druckrohr (Durchmesser 14 mm, Länge 70 cm) gebracht, das mit einem Ölthermostat beheizt wurde. Das Zwischenvolumen der Katalysatorschicht wurde mit feinem Seesand (0,2 bis 0,3 mm) ausgefüllt.

Bei 270 bar und 112°C wurde ein Gemisch aus 1 Gew.-Teil MDA und 1 Gew.-Teil tert.-Butanol zusammen mit Wasserstoff von oben auf den Katalysator geleitet. Die Flüssigkeit rieselte über den Katalysator nach unten in einen Abscheider. Am Kopf des Abscheiders wurden stündlich 30 bis 50 l Wasserstoff entspannt. Der MDA-Durchsatz entsprach einer Katalysatorbelastung von 0,11 bis 0,13 g MDA/ml Kat. x h und wurde in diesem Bereich gehalten.

Nach 333 Stunden zeigte das Reaktionsprodukt laut GC-Analyse folgende Zusammensetzung:

| | |
|---|---|
| trans-trans-Isomeres: | 18,2 % |
| 3- und 4-Kern-Verbindungen: | 0,25 % |
| Di-(4-aminocyclohexyl)-methan | 98,9% |
| Nebenprodukte | 0,85 %. |

## Patentansprüche

1. Verfahren zur Herstellung von Di-(4-aminocyclohexyl)-methan mit einem Anteil von 15 bis 25 Gew.-% des trans-trans-Isomeren durch katalytische Hydrierung von Di-(4-aminophenyl)-methan bei erhöhter Temperatur und erhöhtem Wasserstoffdruck in Gegenwart eines Ruthenium enthaltenden Trägerkatalysators, dadurch gekennzeichnet, daß man einen Ruthenium-Katalysator auf einem mit Verbindungen von seltenen Erdmetallen (SE) und von Mangan behandelten $Al_2O_3$-Träger mit Rutheniumgehalten von 0,05 bis 5 Gew.-%, bevorzugt 0,05 bis 3 Gew.-%, besonders bevorzugt 0,1 bis 2 Gew.-%, bezogen auf das Gesamtgewicht des Katalysators, einsetzt.

2. Verfahren nach Anspruch 1, dadurch gekennzeichnet, daß der Katalysatorträger so mit Verbindungen der SE und des Mangans behandelt wird, daß deren Gehalt zusammen 0,05 bis 8 Gew.-%, bevorzugt 0,2 bis 5 Gew.-%, bezogen auf das Gesamtgewicht des Katalysators, beträgt und das Gewichtsverhältnis SE:Mn auf 5:1 bis 1:5, bevorzugt 10:9 bis 1:2, eingestellt wird.

3. Verfahren nach Anspruch 1, dadurch gekennzeichnet, daß der mit SE und Mn beaufschlagte Katalysatorträger auf eine Temperatur von 200 bis 450°C, bevorzugt 250 bis 430°C, erhitzt wird und danach das Ruthenium aufgetragen wird.

4. Verfahren nach Anspruch 1, dadurch gekennzeichnet, daß als SE eines oder mehrere aus der Gruppe Yttrium, Lanthan, Cer, Praseodym, Neodym und Dysprosium eingesetzt werden, bevorzugt Cer und/oder Lanthan eingesetzt werden, besonders bevorzugt Cer eingesetzt wird.

5. Verfahren gemäß Anspruch 1, dadurch gekennzeichnet, daß der Katalysator ein Oxid, Hydroxid oder Carbonat eines Alkalimetalls enthält.

6. Verfahren nach Anspruch 1, dadurch gekennzeichnet, daß als $Al_2O_3$ die α- oder die γ-Modifikation, bevorzugt die γ-Modifikation, eingesetzt wird.

7. Verfahren nach Anspruch 1, dadurch gekennzeichnet, daß der Katalysator vor der Hydrierung von Di-(4-aminophenyl)-methan durch Behandlung mit Wasserstoff bei 150 bis 350°C aktiviert wird.

8. Verfahren nach Anspruch 1, dadurch gekennzeichnet, daß bei einer Temperatur im Bereich von 80 bis 160°C, bevorzugt 90 bis 140°C, und bei einem Wasserstoffdruck im Bereich von 20 bis 500 bar, bevorzugt 200 bis 400 bar, gearbeitet wird.

9. Verfahren nach Anspruch 1, dadurch gekennzeichnet, daß bei kontinuierlicher Reaktionsführung eine Katalysatorbelastung von 0,05 bis 0,5 kg Di-(4-aminophenyl)-methan pro Liter Katalysator und Stunde eingestellt wird.

10. Verfahren nach Anspruch 1, dadurch gekennzeichnet, daß das zu hydrierende Di-(4-aminophenyl)-methan im Gemisch mit einem Lösungs-/Verdünnungsmittel im Gewichtsverhältnis 1:0,2 bis 1:2, bevorzugt 1:0,5 bis 1:1, eingesetzt wird, wobei als Lösungs-/Verdünnungsmittel Dioxan, Tetrahydrofuran, Isobutanol, tert.-Butanol oder entstehendes Hydriergemisch, bevorzugt das entstehende Hydriergemisch, verwendet werden.

## Claims

1. Process for the preparation of di-(4-aminocyclohexyl)methane containing 15 to 25% by weight of the trans/trans isomer by catalytic hydrogenation of di-(4-aminophenyl)methane at elevated temperature and elevated hydrogen pressure in the presence of a ruthenium-containing supported catalyst, characterised in that a ruthenium catalyst on an $Al_2O_3$ support treated with compounds of rare earth metals (RE) and of manganese and having ruthenium contents of 0.05 to 5% by weight, preferably 0.05 to 3% by weight, particularly preferably 0.1 to 2% by weight, relative to the total weight of the catalyst, is used.

2. Process according to Claim 1, characterised in that the catalyst support is treated with compounds of RE and of manganese in such a manner that together their content is 0.05 to 8% by weight, preferably 0.2 to 5% by weight, relative to the total weight of the catalyst, and the RE/Mn weight ratio is set to 5:1 to 1:5, preferably 10:9 to 1:2.

3. Process according to Claim 1, characterised in that the catalyst support loaded with RE and Mn is heated to a temperature of 200 to 450°C, preferably 250 to 430°C, and the ruthenium is then applied.

4. Process according to Claim 1, characterised in that the RE used is one or more from the group consisting of yttrium, lanthanum, cerium, praseodymium, neodymium and dysprosium, preferably cerium and/or lanthanum, particularly preferably cerium.

5. Process according to Claim 1, characterised in that the catalyst contains an oxide, hydroxide or carbonate of an alkali metal.

6. Process according to Claim 1, characterised in that the $Al_2O_3$ used is the $\alpha$ modification or $\gamma$ modification, preferably the $\gamma$ modification.

7. Process according to Claim 1, characterised in that the catalyst, before hydrogenation of di-(4-aminophenyl)methane, is activated by treatment with hydrogen at 150 to 350°C.

8. Process according to Claim 1, characterised in that the reaction is carried out at a temperature in the range from 80 to 160°C, preferably 90 to 140°C, and at a hydrogen pressure in the range from 20 to 500 bar, preferably 200 to 400 bar.

9. Process according to Claim 1, characterised in that, when the reaction is carried out continuously, the space velocity over the catalyst is set to 0.05 to 0.5 kg of di-(4-aminophenyl)methane per litre of catalyst per hour.

10. Process according to Claim 1, characterised in that the di-(4-aminophenyl)methane to be hydrogenated is used in a mixture with a solvent/diluent in a weight ratio of 1:0.2 to 1:2, preferably 1:0.5 to 1:1, the solvent/diluent used being dioxane, tetrahydrofuran, isobutanol, tert.-butanol or the resulting hydrogenation mixture, preferably the resulting hydrogenation mixture.

## Revendications

1. Procédé de production de di-(4-aminocyclohexyl)méthane contenant une proportion de 15 à 25 % en poids de l'isomère trans-trans par hydrogénation catalytique de di-(4-amiophényl)méthane à une température élevée et sous une pression élevée d'hydrogène en présence d'un catalyseur contenant du ruthénium, fixé sur un support, caractérisé en ce qu'on utilise un catalyseur au ruthénium sur un support en $Al_2O_3$ traité avec des composés de métaux des terres rares (TR) et de manganèse, ayant des teneurs en ruthénium de 0,05 à 5 % en poids, de préférence de 0,05 à 3 % en poids, notamment de 0,1 à 2 % en poids par rapport au poids total du catalyseur.

2. Procédé suivant la revendication 1, caractérisé en ce que le support du catalyseur est traité avec des composés de TR et de manganèse de manière que leurs teneurs s'élèvent conjointement à 0,05-8 % en poids, de préférence à 0,2-5 % en poids par rapport au poids total du catalyseur, et on ajuste le rapport en poids TR : Mn à une valeur de 5:1 à 1:5, de préférence de 10:9 à 1:2.

3. Procédé suivant la revendication 1, caractérisé en ce que le support de catalyseur chargé de TR et de Mn est chauffé à une température de 200 à 450°C, de préférence de 250 à 430°C, et le ruthénium est appliqué ensuite.

4. Procédé suivant la revendication 1, caractérisé en ce qu'on utilise comme TR un ou plusieurs éléments du groupe comprenant l'yttrium, le lanthane, le cérium, le praséodyme, le néodyme et le dysprosium, de préférence le cérium et/ou le lanthane, notamment le cérium.

5. Procédé suivant la revendication 1, caractérisé en ce que le catalyseur contient un oxyde, hydroxyde ou carbonate d'un métal alcalin.

6. Procédé suivant la revendication 1, caractérisé en ce qu'on utilise comme $Al_2O_3$ la forme $\alpha$ ou la forme $\gamma$, de préférence la forme $\gamma$.

7. Procédé suivant la revendication 1, caractérisé en ce que le catalyseur, avant l'hydrogénation du di-(4-aminophényl)méthane, est activé par traitement à l'hydrogène à 150-350°C.

8. Procédé suivant la revendication 1, caractérisé en ce qu'on opère à une température comprise dans la plage de 80 à 160°C, de préférence de 90 à 140°C et à une pression d'hydrogène comprise dans la plage de 20 à 500 bars, de préférence de 200 à 400 bars.

9. Procédé suivant la revendication 1, caractérisé en ce que lorsqu'on conduit la réaction en continu, on ajuste une vitesse spatiale du catalyseur de 0,05 à 0,5 kg de di-(4-aminophényl)méthane par litre de catalyseur et par heure.

10. Procédé suivant la revendication 1, caractérisé en ce que le di-(4-aminophényl)méthane à hydrogéner est utilisé en mélange avec un solvant/diluant dans un rapport de poids de 1:0,2 à 1:2, de préférence de 1:0,5 à 1:1, en utilisant comme solvant/diluant le dioxanne, le tétrahydrofuranne, l'isobutanol, le tertio-butanol ou le mélange d'hydrogénation produit, de préférence le mélange d'hydrogénation produit.